# FASCICULE DE BREVET EUROPEEN

(11) **EP 1 092 401 B1**
(45) Date de publication et mention de la délivrance du brevet: **19.12.2007**
(21) Numéro de dépôt: 00402764.5
(22) Date de dépôt: 06.10.2000
(51) Int. Cl.: A61F 2/06

(54) **Stent en hélice**
Spiralstent
Helical stent

(30) Priorité: 11.10.1999 FR 9912630
(43) Date de publication de la demande: 18.04.2001
(73) Titulaire: B. Braun Medical Société Anonyme, 92100 Boulogne Billancourt (FR)
(72) Inventeur: Nadal, Guy, 86000 Poitiers (FR); Roussigne, Maurice, 86000 Poitiers (FR)
(74) Mandataire: Lerner, François

(56) Documents cités:
- EP-A- 0 884 029
- WO-A-98/40035
- WO-A-98/42277
- FR-A- 2 774 278

## Description

L'invention concerne un implant médical présentant un axe longitudinal principal et prévu pour être introduit dans un conduit anatomique pour traiter une affection dudit conduit, l'implant étant adapté pour occuper un premier état radialement resserré ou un second état radialement déployé, et présentant une extrémité proximale et une extrémité distale, suivant son axe principal.

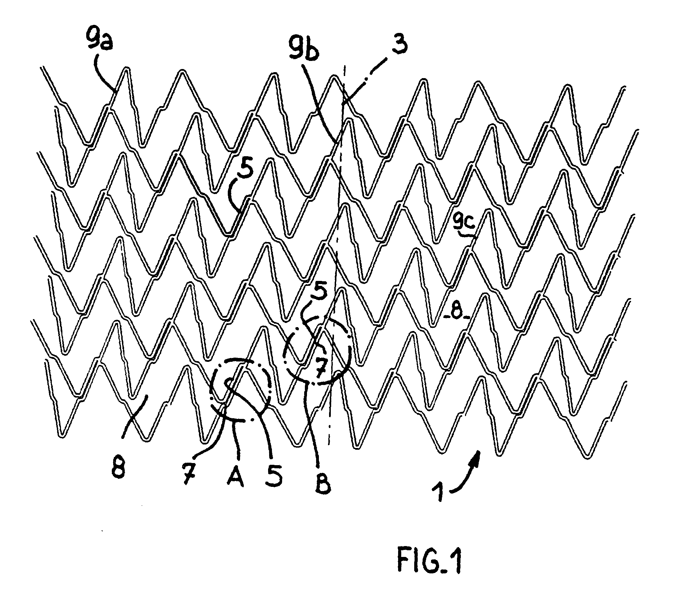

Un exemple d'un tel implant est représenté sur la figure 1, en tant qu'illustration de l'art antérieur.

Sur cette figure, on voit une partie d'une structure de paroi tubulaire d'un implant médical 1 intravasculaire, développé à plat.

Dans cette illustration, la structure présente la forme qu'elle a dans l'état radialement déployé de l'implant. L'axe 3 matérialise la direction longitudinale de l'implant autour de laquelle il se développe suivant sensiblement un tube couramment cylindrique.

On remarque en 5 qu'un tronçon de la structure s'étend à proximité immédiate et sensiblement parallèlement à un autre tronçon 7 qu'il vient en quelque sorte doubler. Cette particularité se reproduit en différents emplacements, tels que ceux repérés A et B sur la figure.

Il s'agit là d'un inconvénient dans la mesure où les méandres sensiblement en zigzags tels que 9a, 9b, 9c paraissent "gauchis". En d'autres termes, dans son état ouvert, la structure de cet implant (également appelé stent) ne paraît pas régulière. En outre, l'existence des tronçons 5 et 7 conduit à une rigidité supérieure du stent en ces endroits à "tronçons doublés", en comparaison des autres tronçons. L'équilibre général de l'implant peut s'en ressentir. Il peut également y avoir une influence sur la fiabilité de l'ouverture, ainsi que sur les caractéristiques mécaniques, voire l'efficacité de l'implant dans son ensemble, compte tenu de la déformation qu'induit cette situation sur les cellules 8 de l'implant.

Le document FR-A-2 744 278 décrit un endoprothèse ayant des éléments structurels de trois types différents, comprenant deux coudes à 180°, une coude à 180° et un coude à 90°, et un seul coude à 90° respectivement. Sauf aux extrémités de l'endoprothèse, chaque raccordement des éléments structurels raccorde un élément du premier type, un élément du second type et un élément du troisième type.

Pour apporter une solution au problème précité et favoriser la qualité globale de l'implant, l'invention concerne un implant médical selon la revendication 1.

Tout d'abord, on peut énoncer que l'invention enseigne de modifier l'implant précité de telle sorte qu'il présente :
- des premiers éléments structurels s'étendant en méandres suivant une série d'hélices parallèles enroulées autour de l'axe longitudinal principal de l'implant, les méandres présentant des sommets ayant des points d'inversion de pente, ces sommets étant dirigés alternativement vers l'extrémité proximale et vers l'extrémité distale,
- des seconds éléments structurels discrets formant des traverses de liaison qui s'étendent individuellement entre deux premiers éléments adjacents qu'elles croisent, ces seconds éléments étant disposés suivant une série de lignes parallèles entre elles et perpendiculaires à l'axe de l'implant,
- lesdits seconds éléments structurels discrets présentant en outre soit uniquement des changements de pente, avec épaulement(s), mais sans inversion de pente, soit des inversions de pente, uniquement, sans épaulement,
- les seconds éléments d'une ligne présentant de préférence un décalage angulaire autour de cet axe vis-à-vis des seconds éléments d'une ligne immédiatement précédente ou suivante.

Une autre manière de présenter la solution principale de l'invention consiste à considérer que les ouvertures ou cellules ménagées dans la paroi essentiellement cylindrique de l'implant présentent, tant dans le premier que dans le second état de l'implant, une forme globalement en "Z" qui est délimitée périphériquement, au moins dans ledit état radialement déployé, par huit sommets où il y a une inversion de pente (ou rebroussement, à la manière par exemple d'un sommet de zigzag).

Une troisième manière de présenter consiste à considérer que l'implant présente des premiers méandres structurels s'étendant essentiellement perpendiculairement à l'axe de l'implant avec des sommets ayant des inversions de pente dirigées alternativement vers l'extrémité proximale puis vers l'extrémité distale, ces sommets étant reliés entre eux par des tronçons intermédiaires de liaison, pour définir une série d'anneaux, plusieurs tels anneaux étant étagés suivant l'axe de l'implant, un premier et un second étages annulaires adjacents qui se succèdent suivant cet axe étant reliés entre eux par des secondes et troisièmes traverses de liaison, discrètes, de formes différentes, croisant les premiers méandres structurels du premier et du second étages, respectivement, ces croisements étant situés à l'endroit de sommets desdits premiers méandres, pour les secondes traverses, et à l'endroit de tronçons intermédiaires de ces mêmes premiers méandres, pour les troisièmes traverses.

En relation avec les première et troisième manières de présenter la solution de l'invention, il est conseillé :
- que les seconds éléments structurels (ou secondes traverses) qui présentent des points d'inversion de pente, mais pas d'épaulement, croisent les premiers éléments (ou méandres) structurels à l'endroit des sommets de ces derniers,
- tandis qu'au contraire, les seconds éléments structurels (ou troisièmes traverses) qui présentent au moins un épaulement, mais pas de zone d'inversion de pente, croiseront avantageusement les premiers éléments, ou méandres, structurels uniquement à l'endroit de certains des tronçons intermédiaires de ces derniers situés entre deux sommets.

Toujours en relation avec les problèmes précités, on notera qu'une autre caractéristique de l'invention conseille que les secondes traverses, c'est-à-dire les seconds éléments structurels discrets sensiblement en "zigzags" (avec sommets, mais sans zone d'épaulement) s'étendent entre les deux premiers éléments, ou premiers méandres, structurels qu'elles relient, sur une partie au moins de la hauteur (ou largeur) occupée par ces derniers, avec pour avantage de contrôler la taille des cellules de l'implant, en particulier dans son état ouvert.

Une description plus détaillée de l'invention va maintenant être fournie en relation avec les dessins annexés dans lesquels :
- la figure 1 montre, en vue développée à plat, une structure d'implant illustrative de l'art antérieur,
- la figure 2 montre un implant (stent) conforme à l'invention, dans son état radialement resserré,
- la figure 3 montre, en vue partielle développée à plat, la forme de la structure de la figure 1, en vue grossie,
- la figure 4 montre une cellule de la structure de la figure 3,
- la figure 5 montre, en vue développée à plat, une partie de l'implant de la figure 2,
- la figure 6 montre la même structure que la figure 3, mais avec renforcement des lignes de méandres perpendiculaires à l'axe longitudinal de l'implant,
- la figure 7 correspond à la figure 5, mais avec les lignes renforcées de la figure 6,
- et les figures 8, 9 et 10 reprennent l'illustration des figures 3 et 6, mais avec un renforcement des lignes de méandres "en hélices".

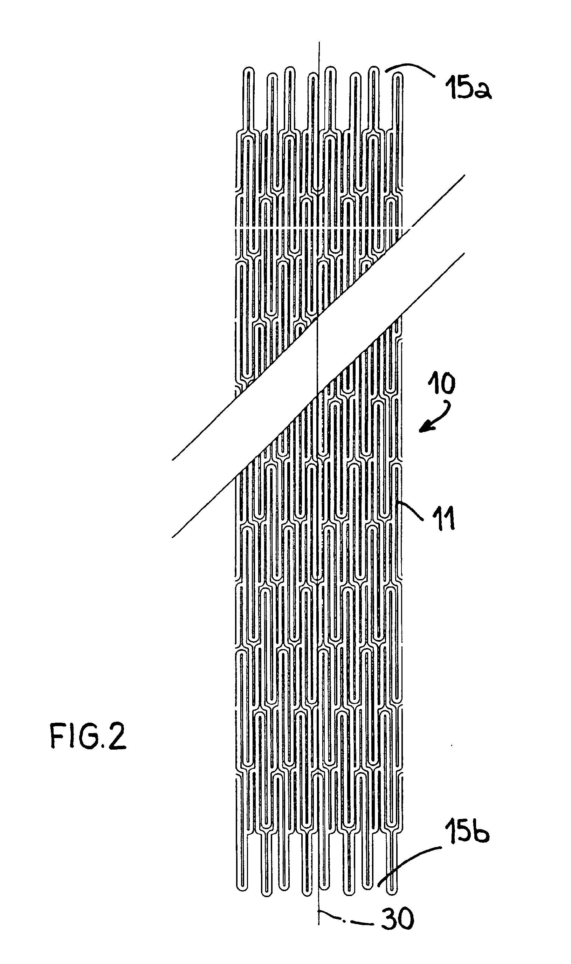

Sur la figure 2, on voit globalement l'aspect d'un stent 10 conforme à l'invention.

Comme couramment employé, le terme "stent" désigne un implant destiné à être introduit dans un conduit naturel d'un corps, tel en particulier qu'un vaisseau. Il s'agit typiquement d'une structure de soutien de la paroi du conduit (vaisseau iliaque notamment) qui peut être utilisée comme élargisseur, tout particulièrement en cas de sténose.

L'implant 10 présente un axe longitudinal 30 autour duquel sa structure 11 s'étend, en présentant une forme tubulaire, cylindrique, que l'on distingue habituellement mieux dans son état radialement déployé.

Sur la figure 2, le stent est dans son état radialement resserré, prêt à être introduit dans le conduit concerné, en particulier par voie endoluminale, par exemple par la technique dite "de SELDINGER".

La structure 11 est de préférence métallique. Elle peut être à base d'acier inoxydable ou en alliage à mémoire de forme thermique (tel que du "Nitinol"®).

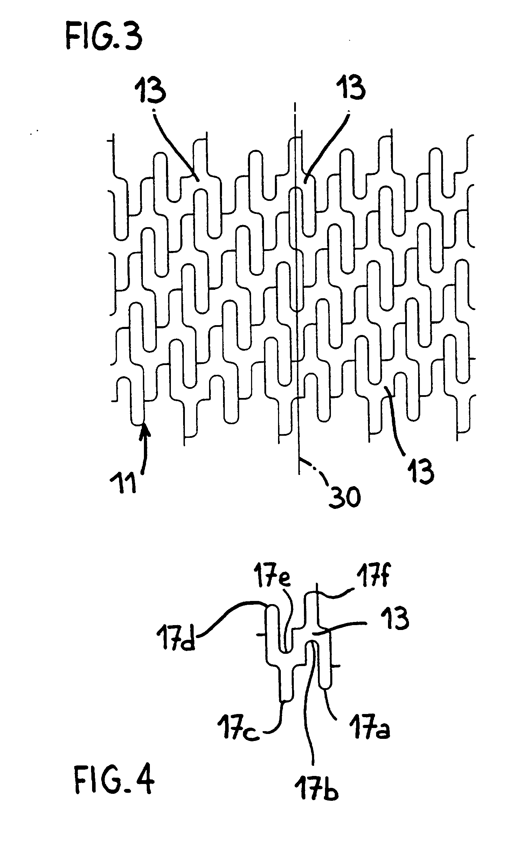

Sur la figure 3, on retrouve en vue agrandie et dans des proportions différentes le dessin de la structure 11 dont on a volontairement écarté les lignes pour le rendre plus lisible.

On remarquera que la structure 11 est monobloc et comporte une succession de cellules, ou "ouvertures" traversantes, 13, dont l'une a été représentée sur la figure 4.

En l'espèce, toutes les cellules de la structure sont identiques (sauf à la limite les cellules des deux extrémités, respectivement proximale, 15a, et distale 15b, où tous les angles sont arrondis).

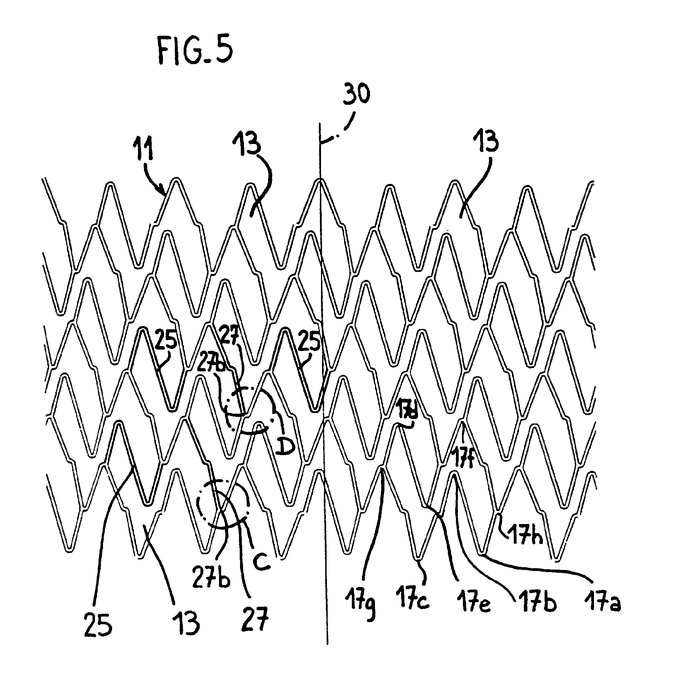

Sur les figures 3 et 4, on remarquera la forme globalement en "Z" ou en "S" de chaque cellule 13, cette forme pouvant être retrouvée sur la figure 5, où l'on voit toujours un détail agrandi de la structure 11, mais dans un état radialement déployé par rapport à l'axe longitudinal 30.

Sur les figures 3 et 4, chaque cellule 13 est périmétriquement délimitée par six sommets 17a, 17b, 17c, 17d, 17e, 17f, c'est-à-dire six zones d'inversion de pente ou encore "de rebroussement", ces six sommets devenant huit dans l'état radialement déployé (figure 5), avec les sommets 17g, 17h, créés par les tronçons de raccordement de la cellule aux cellules adjacentes et dont on voit les amorces sur la figure 4.

Cette forme de cellules en "Z" présente un avantage certain par rapport à la forme des cellules de la figure 1 et qui est moins régulière, compte tenu de l'existence du tronçon 5.

Sur la figure 5, on peut constater que ce tronçon a disparu, comme montré aux emplacements C et D, ce qui permet une ouverture radiale plus équilibrée de l'implant, en particulier.

Par rapport aux cellules de la figure 1, celles de l'implant de l'invention comprennent au moins un sommet de moins.

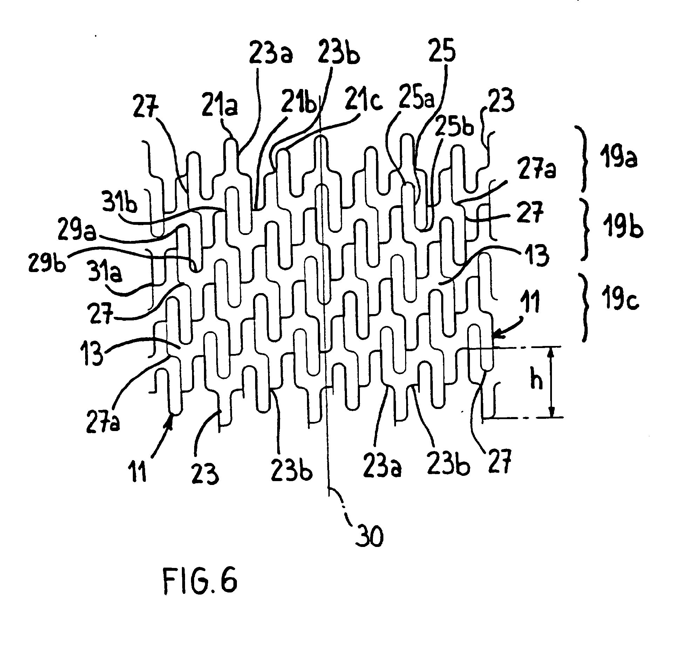

Sur la figure 6, on a renforcé le trait des méandres constitués par les lignes structurelles que l'on voit ici développées à plat et qui s'étendent donc perpendiculairement à l'axe longitudinal 30, en définissant, le long de l'axe 30, une série d'étages annulaires (une fois ces lignes refermées chacune sur elles-mêmes) tels que 19a, 19b, 19c.

Les méandres de chaque étage (que l'on retrouve à l'identique d'étage en étage, à la seule différence près, aux deux extrémités, que tous les sommets sont plus arrondis) sont constitués d'une succession de sommets tels que 21a, 21b, 21c, réunis successivement deux à deux par des tronçons intermédiaires tels que 23a, 23b.

On appellera ces méandres des "premiers méandres structurels" référencés 23.

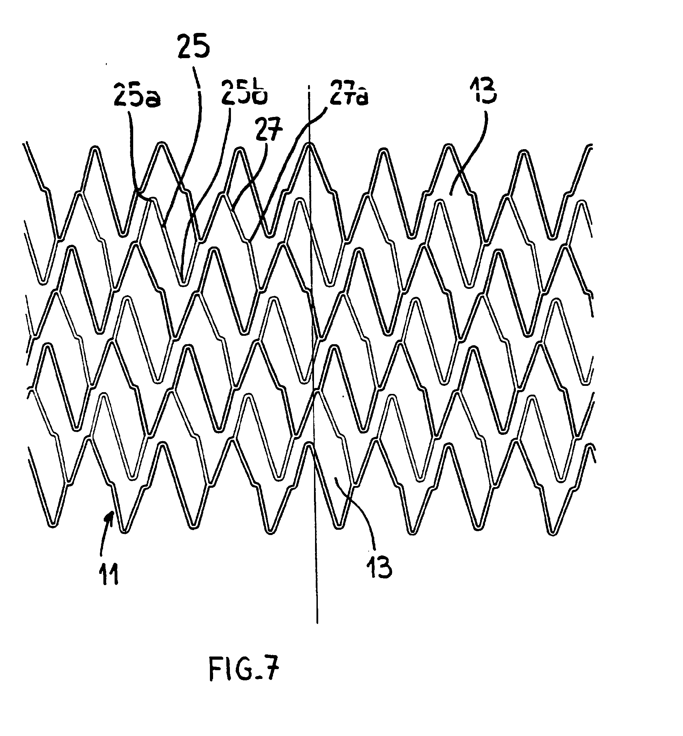

Sur les figures 6 et 7, on peut constater que lesdits méandres de deux étages adjacents, tels que 19a, 19b, sont réunis entre eux par des secondes et des troisièmes traverses de liaison 25 et 27, respectivement.

Tant dans l'état radialement resserré que dans l'état radialement déployé, les secondes et troisièmes traverses, qui sont courbes, présentent exclusivement comme zones de courbure, deux sommets inversés 25a, 25b, et un épaulement 27a, respectivement.

Ainsi, les secondes traverses 25 présentent uniquement des tronçons rectilignes entre leurs deux sommets, 25a, 25b, où la pente de la traverse s'inverse sensiblement en épingle à cheveux, successivement dans un sens et dans l'autre.

Par contre, chaque troisième traverse 27 présente, sensiblement à mi-longueur, un épaulement arrondi, avec donc changement de pente, mais sans inversion.

Pour obtenir la forme sensiblement en "Z" des cellules 13, les secondes traverses 25 (qui ont donc une forme sensiblement en "S") sont raccordées, a chacune de leurs extrémités, à l'endroit de sommets opposés des méandres constituant les deux étages adjacents considérés, tandis que les extrémités des troisièmes traverses 27 sont raccordées en des zones intermédiaires entre deux sommets successifs d'un même étage.

On aura compris que l'on appelle "sommets opposés", deux sommets dirigés, pour l'un, vers l'extrémité proximale et, pour l'autre, vers l'extrémité distale de la structure.

Dans l'état resserré, aucun sommet n'est d'ailleurs dirigé autrement que vers l'extrémité distale ou proximale de l'implant.

Deux lignes en méandres définissant deux étages adjacents tels que 19a, 19b, sont par ailleurs décalées circonférenciellement l'une par rapport à l'autre, avec un décalage de phase partiel.

On notera également que les raccordements des secondes traverses 25 s'effectuent entre sommets "extrêmes" des méandres de deux étages adjacents, c'est-à-dire avec des sommets qui, dans un sens ou en sens opposé, sont les plus "hauts" des méandres, à la différence par exemple des sommets "moins hauts" tels que les sommets arrondis 29a, 29b, de part et d'autre desquels se raccordent des troisièmes traverses 27, en zone intermédiaire entre l'un de ces sommets et un sommet "haut" suivant, tel que 31b, ou précédent, tel que 31a.

Concernant les troisièmes traverses 27, on notera comme le voit peut-être plus distinctement aux endroits renforcés en gras, que la forme des troisièmes traverses 27, en liaison avec le raccordement de leur extrémité inférieure 27b, favorise bien l'équilibre général de l'implant et autorise l'obtention des cellules 13 en "Z" précitées, ceci en conjonction avec la forme et les points d'attache des secondes traverses 25, comme on le peut le voir en particulier sur la figure 5.

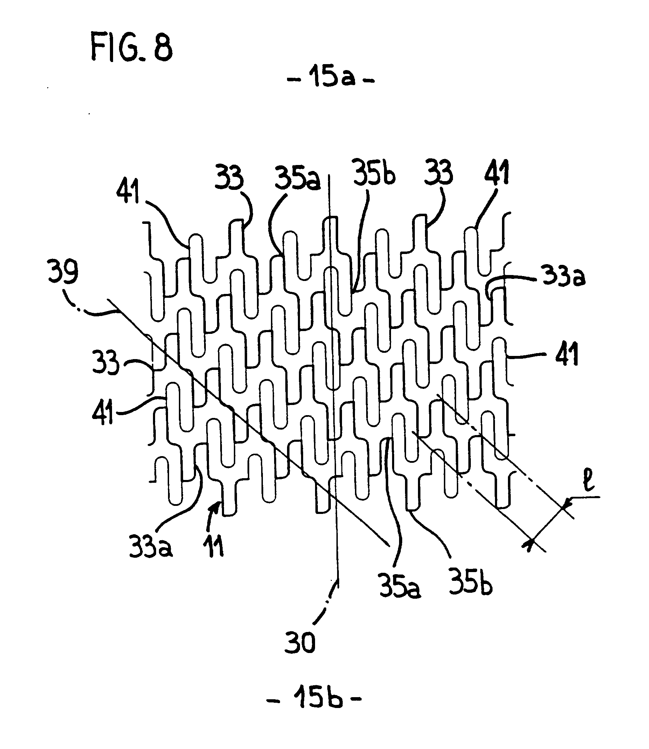

Si l'on ne raisonne plus en termes de "cellules", ni en termes d'étages annulaires sensiblement perpendiculaires à l'axe 30, mais plutôt avec une approche longitudinale, la vision que l'on a de la structure 11 de l'implant est encore différente, comme en témoignent les figures 8, 9 et 10 où l'on a renforcé le trait des méandres qui se développent, en hélices, parallèlement les uns aux autres autour de l'axe 30.

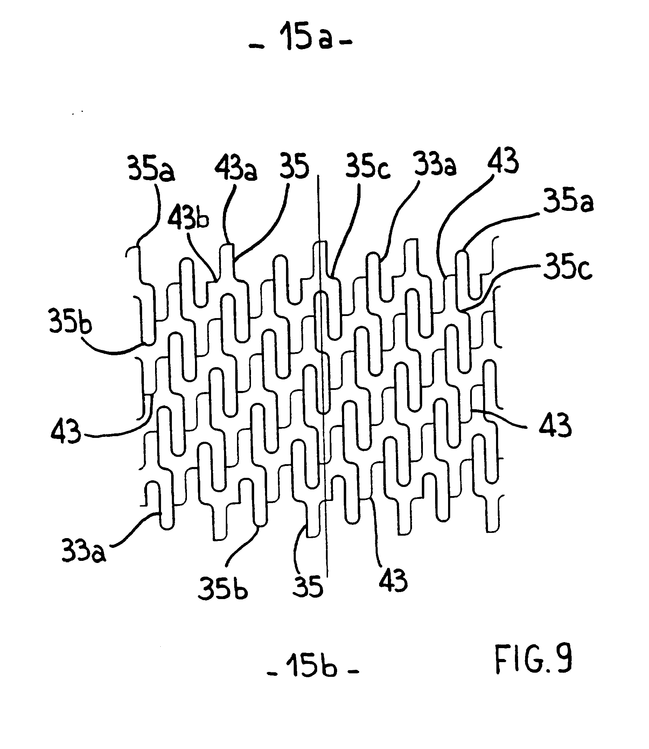

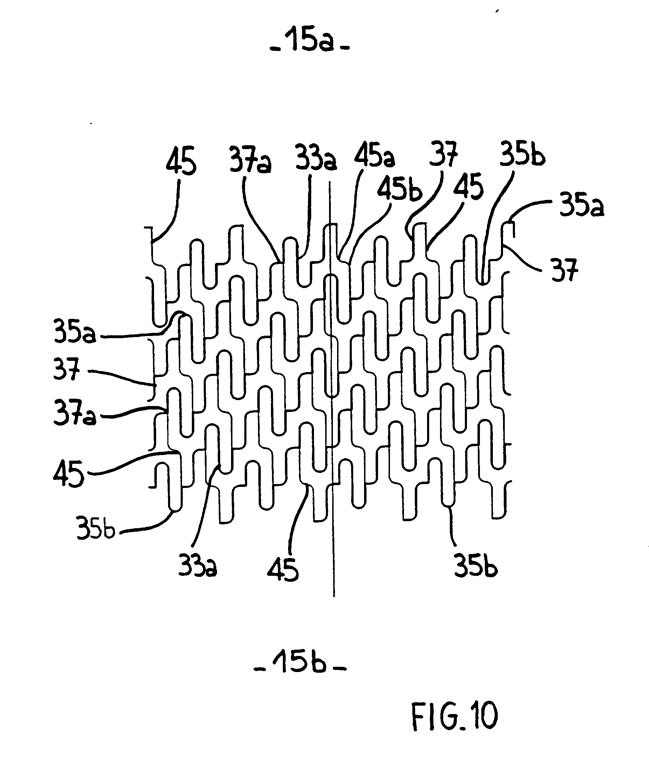

Sur ces figures, on a repéré successivement ces lignes de méandres, 33 (figure 8), 35 (figure 9) et 37 (figure 10).

Sur la figure 8, on a en outre repéré en 39 l'un des axes en hélice (une fois la structure refermée sur elle-même) le long duquel se développe l'une des lignes en méandres 33.

Ces méandres en hélices 33 seront dénommées "premiers éléments structurels". Comme les méandres précédents, tels que 23, ils présentent une succession de sommets reliés entre eux, deux à deux, par des tronçons intermédiaires dont certains présentent un épaulement et d'autres non (voir tronçon 33a).

Bien entendu, pour former les méandres, les sommets successifs sont opposés, tels que 35a dirigé vers l'extrémité 15a, puis 35b dirigé vers l'extrémité 15b.

On notera une fois encore le décalage angulaire partiel des méandres sur la circonférence de la structure.

Les premiers éléments structurels 33 sont en outre reliés deux à deux, d'une ligne à une ligne immédiatement adjacente, par des seconds éléments structurels discrets qui intersectent les premiers, pour constituer la structure monobloc recherchée.

Sur les figures 8, 9 et 10, on a successivement repéré ces seconds éléments 41, 43 et 45.

Sur la figure 8, ces seconds éléments 41 présentent une forme en "S" (basculée à 90°) à deux sommets arrondis sensiblement en épingle à cheveux (du moins dans l'état radialement resserré représenté de la structure), à l'image des "secondes traverses" 25 de la figure 6 (ce sont d'ailleurs les mêmes tronçons filamentaires). Ces tronçons 41 se raccordent à des sommets inverses (tels que 35a, 35b) de deux lignes de méandres 33 adjacentes.

Sur la figure 9, les tronçons 43 présentent une forme de "S" déformée, sans sommet, avec deux coudes successifs inversés, arrondis sensiblement à 90°, et repérés 43a, 43b, pour l'un des tronçons.

Sur la figure 10, on retrouve encore cet aspect sensiblement de "S" déformé avec deux coudes successifs arrondis sensiblement à 90°, repérés 45a, 45b, pour ces deux éléments structurels discrets 45 (la forme du "S" est basculée de 90° verticalement, comme sur la figure 8).

Les "seconds éléments structurels" 43 et 45 se raccordent à des zones de méandres, respectivement 35, 37, situées entre deux sommets inverses successifs de chacun de ces derniers.

Pour chaque élément 43, le raccordement s'effectue, à l'une et l'autre de ses extrémités, entre un sommet et un épaulement intermédiaire (tel que 35c) de chacune des deux lignes en méandres 35 concernées.

Pour chaque élément 45, le raccordement des extrémités s'effectue à l'endroit d'épaulements 37a présents sur les deux lignes successives en méandres 37 concernées.

Une constante à remarquer sur les figures 8 à 10 ; on ne trouve pas d'élément structurel discret entre les méandres continus en hélices (33, 35, 37) qui présente à la fois des zones d'épaulement (avec donc un ressaut arrondi; tels que 43a, 43b ; 45a, 45b) et des sommets (avec donc une inversion de pente ; tronçon 41), comme on en trouve dans l'art antérieur de la figure 1.

On pourra également remarquer que les seconds éléments discrets précités 41, 43, 45, s'étendent suivant une série de lignes parallèles entre elles qui forment également des hélices s'enroulant autour de l'axe 30.

Lesdits seconds éléments structurels pourraient également être présentés comme organisés suivant une série de lignes toujours parallèles encre elles, mais perpendiculaires à l'axe 30, avec un décalage angulaire circonférenciel entre deux lignes adjacentes (disposition sensiblement en quinconce).

Sur les figures 6 et 8, on a référencé, respectivement, 1 la largeur d'une ligne de méandres 33 et h la hauteur (parallèlement à l'axe 30) d'une ligne de méandres 23, ceci pour montrer que, dans un cas comme dans l'autre, des secondes traverses de liaison (respectivement 41 et 27) s'étendent, entre deux tels éléments en méandres qu'elles relient, sur une partie de la largeur (respectivement hauteur) occupée par ces méandres. Une conséquence de cela est que l'on obtient des cellules de taille adaptée, avec la forme souhaitée, en évitant notamment le gauchissement de l'illustration de la figure 1.

## Revendications

1. Implant médical présentant un axe longitudinal principal (30) et prévu pour être introduit dans un conduit anatomique pour traiter une affection dudit conduit, l'implant étant adapté pour occuper un premier état radialement resserré ou un second état radialement déployé, l'implant présentant :
- une extrémité proximale (15a) et une extrémité distale (15b), suivant l'axe principal,
- des premiers éléments structurels (33, 35, 37) s'étendant en méandres suivant une série d'hélices parallèles enroulées autour de l'axe longitudinal principal de l'implant, les méandres présentant des sommets (35a, 35b) ayant des points d'inversion de pente, ces sommets étant dirigés alternativement vers l'extrémité proximale et vers l'extrémité distale,
- des seconds éléments structurels discrets (41, 43, 45) formant des traverses de liaison qui s'étendent individuellement entre deux premiers éléments adjacents qu'elles croisent, ces seconds éléments étant disposés suivant une série de lignes parallèles entre elles et perpendiculaires à l'axe de l'implant,
- lesdits seconds éléments structurels discrets présentant en outre soit uniquement des changements de pente (45a, 45b), avec épaulement(s) mais sans inversion de pente, soit des inversions de pente (41), uniquement, sans épaulement.

2. Implant médical selon la revendication 1, **caractérisé en ce que** les seconds éléments (41, 43, 45) présentent deux courbures, l'une inverse de l'autre, de telle sorte qu'ils ont individuellement une forme sensiblement en "S".

3. Implant médical selon la revendication 1 ou la revendication 2, **caractérisé en ce que** :
- lesdits seconds éléments structurels (41) qui présentent des points d'inversion de pente, mais pas d'épaulement, croisent les premiers éléments structurels (33) à l'endroit de sommets (35a, 35b) de ces derniers,
- tandis que les seconds éléments structurels (43, 45) qui présentent au moins un épaulement (45a, 45b), mais pas de zone d'inversion de pente, croisent les premiers éléments structurels (35, 37) uniquement à l'endroit de certains des tronçons intermédiaires de ces derniers situés entre deux sommets successifs.

4. Implant médical selon l'une quelconque des revendications précédentes, **caractérisé en ce que** tous les éléments structurels forment des ouvertures (13) qui sont identiques ou quasi identiques.

## Claims

1. Medical implant which has a main longitudinal axis (30) and which is intended to be introduced into a bodily passage in order to treat an illness of said passage, the implant being capable of occupying a first radially contracted state or a second radially deployed state, the implant having:
- a proximal end (15a) and a distal end (15b) along the main axis,
- first structural elements (33, 35, 37) which extend in a meandering manner along a series of parallel helices which extend around the main longitudinal axis of the implant, the meandering portions having peaks (35a, 35b) which have inclination inversion locations, these peaks being directed alternately towards the proximal end and the distal end,
- separate second structural elements (41, 43, 45) which form connection cross-members which extend individually between two first adjacent elements with which they intersect, these second elements being arranged in a series of mutually parallel lines which are perpendicular relative to the axis of the implant,
- the separate second structural elements further having either only changes of inclination (45a, 45b) with shoulder(s) but with no inversion of inclination, or only inclination inversions (41), with no shoulder.

2. Medical implant according to claim 1, **characterised in that** the second elements (41, 43, 45) have two curves, one being the inverse of the other, so that individually they are substantially S-shaped.

3. Medical implant according to claim 1 or claim 2,
**characterised in that**:
- the second structural elements (41), which have locations of inclination inversion but no shoulder, intersect with the first structural elements (33) at the location of peaks (35a, 35b) thereof,
- whilst the second structural elements (43, 45), which have at least one shoulder (45a, 45b) but no zone of inclination inversion, intersect with the first structural elements (35, 37) only at the location of some of the intermediate portions thereof located between two successive peaks.

4. Medical implant according to any one of the preceding claims, **characterised in that** all the structural elements form openings (13) which are identical or almost identical.

## Patentansprüche

1. Medizinisches Implantat, das eine Hauptlängsachse (30) aufweist und dazu bestimmt ist, in einen anatomischen Gang eingeführt zu werden, um eine Erkrankung dieses Ganges zu behandeln, wobei das Implantat dazu geeignet ist, einen ersten radial eingeengten und einen zweiten radial entfalteten Zustand einzunehmen, wobei das lmplantat aufweist:
- entlang der Hauptachse ein proximales Ende (15a) und ein distales Ende (15b),
- erste Strukturelemente (33, 35, 37), die sich als Mäander entlang einer Reihe von parallelen Spiralen, welche um die Hauptlängsachse des Implantats gerollt sind, erstrecken, wobei die Mäander Scheitel (35a, 35b) mit umgekehrten Neigungspunkten haben, und diese Scheitel alternativ zu dem proximalen Ende und dem distalen Ende hin gerichtet sind,
- zweite diskrete Strukturelemente (41, 43, 45), welche Verbindungstraversen bilden, die sich individuell zwischen zwei ersten nebeneinander liegenden Elementen, welche sie kreuzen, erstrecken, wobei diese zweiten Elemente entlang einer Reihe von Linien angeordnet sind, die untereinander parallel und senkrecht zu der Implantatachse sind,
- wobei die zweiten diskreten Strukturelemente ferner entweder alleinig Neigungsänderungen (45a, 45b) mit Schulter(n) aber ohne Neigungsumkehrung, oder Neigungsumkehrungen (41) alleinig ohne Schulter aufweisen.

2. Medizinisches Implantat nach Anspruch 1, **dadurch gekennzeichnet, daß** die zweiten Elemente (41, 43, 45) zwei Krümmungen aufweisen, die eine zu der anderen umgekehrt, derart, daß sie einzeln im wesentlichen eine "S"-Form haben.

3. Medizinisches lmplantat nach Anspruch 1 oder nach Anspruch 2, **dadurch gekennzeichnet, daß**:
- die zweiten Strukturelemente (41), welche umgekehrte Neigungspunkte, aber keine Schulter aufweisen, die ersten Strukturelemente 33 an der Stelle der Scheitel (35a, 35b) dieser letzteren kreuzen,
- während die zweiten Strukturelemente (43, 45), welche mindestens eine Schulter (45a, 45b), aber keine Neigungsumkehrzone aufweisen, die ersten Strukturelemente (35, 37) einzig an der Stelle bestimmter Zwischenabschnitte dieser letzteren kreuzen, welche zwischen zwei aufeinander folgenden Scheiteln liegen.

4. Medizinisches Implantat nach einem der vorhergehenden Ansprüche, **dadurch gekennzeichnet, daß** alle Strukturelement Öffnungen (13) bilden, die identisch oder quasi identisch sind.
